(19) Europäisches Patentamt
European Patent Office
Office européen des brevets

(11) Veröffentlichungsnummer: **0 200 064 A1**

# (12) EUROPÄISCHE PATENTANMELDUNG

(21) Anmeldenummer: 86105084.7

(51) Int. Cl.[4]: C 07 D 251/22

(22) Anmeldetag: 14.04.86

(30) Priorität: 27.04.85 DE 3515285

(43) Veröffentlichungstag der Anmeldung: 05.11.86 Patentblatt 86/45

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Riebel, Hans-Jochem, Dr.
In der Beek 92
D-5600 Wuppertal 1(DE)

(72) Erfinder: Fauss, Rudolf, Dr.
Gerstenkamp 10
D-5000 Köln 80(DE)

(72) Erfinder: Krebs, Andreas, Dr.
Am Gartenfeld 70
D-5068 Odenthal-Holz(DE)

## (54) Verfahren zur Herstellung von 2-Cyanamino-4,6-dialkyl-1,3,5-triazinen.

(57) Die Erfindung betrifft ein neues Verfahren zur Herstellung von 2-Cyanamino-4,6-dialkyl-1,3,5-triazinen der allgemeinen Formel (I)

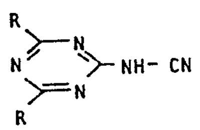 (I)

in welcher
R für Alkyl steht,
das dadurch gekennzeichnet ist, daß man 2-Chlor-4, 6-dialkyl-1,3,5-triazine der Formel (II)

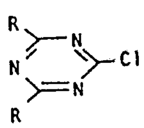 (II)

in welcher
R die oben angegebenen Bedeutungen hat,
mit Cyanamiden der Formel (III)

$(Me)_2N-CN$ (III)

in welcher
Me für ein Äquivalent eines Alkali- oder Erdalkalimetallions steht,
in Gegenwart von Wasser bei Temperaturen zwichen 0 °C und 35 °C umsetzt.

EP 0 200 064 A1

BAYER AKTIENGESELLSCHAFT 5090 Leverkusen-Bayerwerk

Konzernverwaltung RP Bi/mö
Patentabteilung IVa/ZP

# Verfahren zur Herstellung von 2-Cyanamino-4,6-dialkyl-1,3,5-triazinen

Die vorliegende Erfindung betrifft ein neues Verfahren zur Herstellung von 2-Cyanamino-4,6-dialkyl-1,3,5-triazinen, welche als Zwischenprodukte für die Herstellung von Herbiziden und Pflanzenwachstumsregulatoren verwendet werden können.

Es ist bereits bekannt, daß 2-Cyanamino-1,3,5-triazine durch Umsetzung von Alkalimetall- oder Erdalkalimetall-Salzen von Cyanamid mit den entsprechenden 2-Halogen-1,3,5-triazinen erhalten werden (vgl. z.B. DE-OS 3 334 455 / EP-A-121 082).
Die Nachteile bei diesem Verfahren sind die relativ hohen Reaktionstemperaturen und die Verwendung von ökologisch nicht unproblematischen inerten Lösungsmitteln.

Es wurde nun gefunden, daß man 2-Cyanamino-4,6-dialkyl-1,3,5-triazine der allgemeinen Formel (I)

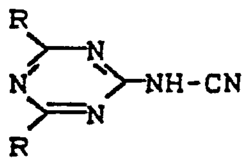

(I)

in welcher

R für Alkyl steht,

erhält, wenn man 2-Chlor-4,6-dialkyl-1,3,5-triazine der Formel (II)

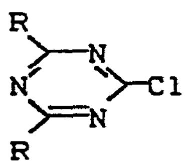

(II)

in welcher

R die oben angegebene Bedeutung hat,

mit Cyanamiden der Formel (III)

$$(Me)_2N\text{-}CN \qquad (III)$$

in welcher

Me für ein Äquivalent eines Alkali- oder Erdalkalimetallions steht,

in Gegenwart von Wasser bei Temperaturen zwischen 0 °C und 35 °C umsetzt.

Überraschenderweise gelingt es mit Hilfe des erfindungsgemäßen Verfahrens unter schonenden Reaktionsbedingungen die Verbindungen der Formel (I) herzustellen. Weitere Vorteile des Verfahrens bestehen darin, daß man Wasser als Verdünnungsmittel einsetzen kann und einen geringeren Energieverbrauch hat.

Bevorzugt werden mit Hilfe des erfindungsgemäßen Verfahrens die Verbindungen der Formel (I) hergestellt,

in welcher

R für Alkyl mit 1 bis 6 Kohlenstoffatomen steht.

Besonders bevorzugt werden diejenigen Verbindungen der Formel (I) hergestellt,

in welcher

R für Methyl, Ethyl, n-Propyl, i-Propyl, n-Butyl, i-Butyl, sec.-Butyl oder tert.-Butyl steht.

Ganz besonders bevorzugt werden diejenigen Verbindungen der Formel (I) hergestellt,

in welcher

R für Methyl, Ethyl, n-Propyl, i-Propyl oder n-Butyl steht.

Verwendet man beispielsweise für das erfindungsgemäße Verfahren 2-Chlor-4,6-diethyl-1,3,5-triazin und Cyanamiddinatriumsalz als Ausgangsstoffe, so kann die Reaktion durch das folgende Formelschema wiedergegeben werden:

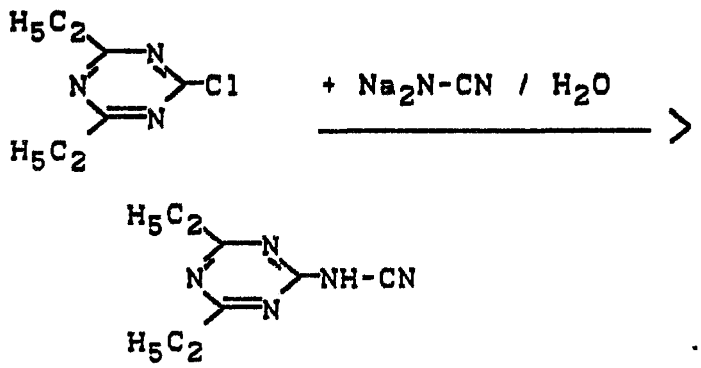


Die als Ausgangsstoffe für das erfindungsgemäße Verfahren zu verwendenden 2-Chlor-4,6-dialkyl-1,3,5-triazine sind durch die Formel (II) allgemein definiert. In dieser Formel steht R bevorzugt für diejenigen Reste, welche oben im Rahmen der Substituentendefinition der Formel (I) vorzugsweise bzw. als insbesondere bevorzugt angegeben sind.

Als Beispiele für die Verbindungen der Formel (II) seien genannt:

4,6-Dimethyl-, 4,6-Diethyl-, 4,6-Di-n-propyl-, 4,6-Di--iso-propyl-, 4,6-Di-n-butyl-, 4,6-Di-iso-butyl-, 4,6--Di-sec.-butyl- und 4,6-Di-tert.-butyl-2-chlor-1,3,5--triazin.

Die Verbindungen der Formel (II) sind bekannt oder lassen sich nach bekannten Verfahren in analoger Weise herstellen.

Die weiterhin als Ausgangsstoffe für das erfindungsgemäße Verfahren zu verwendenden Cyanamide sind durch die Formel (III) allgemein definiert. In dieser Formel steht Me vorzugsweise für ein Natrium-, Kalium- oder Calciumäquivalent.

Als Beispiele für die Verbindungen der Formel (III) seien genannt:

Dinatriumcyanamid, Dikaliumcyanamid und Calciumcyanamid.

Die Verbindungen der Formel (III) sind allgemein bekannte Verbindungen der organischen Chemie.

Das erfindungsgemäße Verfahren wird in Gegenwart von Wasser als Verdünnungsmittel durchgeführt.

Das erfindungsgemäße Verfahren wird im allgemeinen bei Temperaturen zwischen 0 °C und 35 °C, vorzugsweise zwischen 0 °C und 25 °C durchgeführt. Die Umsetzungen werden im allgemeinen bei Normaldruck durchgeführt.

Bei der Durchführung des erfindungsgemäßen Verfahrens werden die Ausgangsstoffe gewöhnlich in äquimolaren Mengen eingesetz. Die Aufarbeitung der Verbindungen der Formel (I) geschieht nach üblichen Methoden. Nach beendeter Zugabe der Ausgangsstoffe wird noch kurze Zeit oder auch mehrere Stunden bei 15°C bis 25°C nachgerührt. Anschließend wird eingeengt und der Rückstand mit Wasser versetzt und mit einer Mineralsäure, wie z.B. Salzsäure angesäuert. Die Verbindungen der Formel (I) fallen im allgemeinen in kristalliner Form an.

Die nach den erfindungsgemäßen Verfahren herzustellenden 2-Cyanamino-4,6-dialkyl-1,3,5-triazine können als Zwischenprodukte für die Herstellung von Guanidin-Derivaten, die als Herbizide und Pflanzenwuchsregulatoren wirksam sind, eingesetzt werden (vgl. EP-OS 121 082).

Herstellungsbeispiel

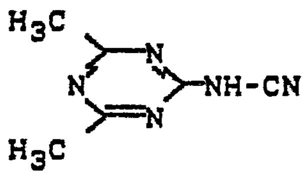

Zu einer Suspension von 114,8 g(0,8 Mol) 2-Chlor-4,6-dimethyl-1,3,5-triazin in 400 ml Eiswasser wird unter starkem Rühren in 4 Stunden eine Lösung von 72,0 g (0,84 Mol) Cyanamiddinatriumsalz in 400 ml Wasser bei einer Temperatur von 0 °C bis 5 °C getropft. Anschließend wird bei 20 °C nachgerührt und 15 bis 16 Stunden stehengelassen. Nach Ansäuern mit konz. Salzsäure auf pH = 2 wird abgesaugt, viermal mit 80 ml Eiswasser gewaschen und getrocknet.

Man erhält 86,1 g (72 % der Theorie) 2-Cyanamino-4,6-dimethyl-1,3,5-triazin vom Schmelzpunkt 241 °C (Zers.).

Patentansprüche

1) Verfahren zur Herstellung von 2-Cyanamino-4,6-dialkyl-1,3,5-triazinen der allgemeinen Formel (I)

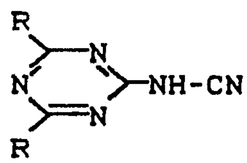 (I)

in welcher

R für Alkyl steht,

dadurch gekennzeichnet, daß man 2-Chlor-4,6-dialkyl-1,3,5-triazine der Formel (II)

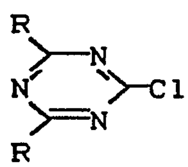 (II)

in welcher

R die oben angegebene Bedeutung hat,

mit Cyanamiden der Formel (III)

$$(Me)_2N\text{-}CN \qquad (III)$$

in welcher

Me für ein Äquivalent eines Alkali- oder Erdalkalimetallions steht,

in Gegenwart von Wasser bei Temperaturen zwischen 0 °C und 35 °C umsetzt.

Europäisches Patentamt

**0200064**

Nummer der Anmeldung

# EUROPÄISCHER RECHERCHENBERICHT

EP 86105084.7

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | GB - A - 1 052 567 (LONZA)<br>* Anspruch 1; Beispiele 1,2 *<br>-- | 1 | C 07 D 251/22 |
| A | CHEMICAL ABSTRACTS, Band 94, Nr. 19, 11. Mai 1981, Columbus, Ohio, USA<br>DOVLATYAN, V.V.; KHACHATRYAN, L.A.; AMBARTSUMYAN, E.N. " Action of acid chlorides, hydrochloric acid, and carbon dioxide on cyanamino-s-triazine salts"<br>Seite 656, Spalte 2, Zusammenfassung-Nr. 156 874q<br>& Arm. Khim. Zh. 1980, 33(9), 755-8<br>---- | 1 | |
| | | | RECHERCHIERTE SACHGEBIETE (Int. Cl.4) |
| | | | C 07 D 251/00 |

Der vorliegende Recherchenbericht wurde für alle Patentansprüche erstellt.

| Recherchenort | Abschlußdatum der Recherche | Prüfer |
|---|---|---|
| WIEN | 16-07-1986 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82